# EUROPÄISCHE PATENTSCHRIFT

(11) **EP 0 731 082 B1**
(45) Veröffentlichungstag und Bekanntmachung des Hinweises auf die Patenterteilung: **02.06.1999**
(21) Anmeldenummer: 96103150.7
(22) Anmeldetag: 01.03.1996
(51) Int. Cl.: C07C 57/04, C07C 51/25, C07C 47/22, C07C 45/35

(54) **Verfahren der kontinuierlich betriebenen heterogen katalysierten Gasphasenoxidation von Propylen zu Acrolein, Acrylsäure oder deren Gemisch**
Process for continuous heterogenous catalyzed partial oxidation of propylene in gaseous phase to acrolein, acrylic acid or their mixture
Procédé d'oxydation partielle catalysée hétérogène du propylène en phase gazeuse en acroléine, en acide acrylique ou leur mélange

(30) Priorität: 10.03.1995 DE 19508532; 13.07.1995 DE 19525506
(43) Veröffentlichungstag der Anmeldung: 11.09.1996
(73) Patentinhaber: BASF AKTIENGESELLSCHAFT, 67056 Ludwigshafen (DE)
(72) Erfinder: Hefner, Werner, Dr., 68623 Lampertheim (DE); Machhammer, Otto, Dr., 67281 Kirchheim (DE); Neumann, Hans-Peter, Dr., 67067 Ludwigshafen (DE); Tenten, Andreas, Dr., 67487 Maikammer (DE); Ruppel, Wilhelm, Dr., 67227 Frankenthal (DE); Vogel, Herbert, Dr., 67069 Ludwigshafen (DE)

(56) Entgegenhaltungen:
- EP-A- 0 253 409
- EP-A- 0 257 565
- EP-A- 0 361 372
- DE-A- 3 002 829

## Beschreibung

Die vorliegende Erfindung betrifft ein neues Verfahren der kontinuierlich betriebenen heterogen katalysierten Gasphasenoxidation von Propylen zu Acrolein, Acrylsäure oder deren Gemisch in einem Oxidationsreaktor, dessen Beschickungsgasgemisch neben Propylen und molekularem Sauerstoff als Oxidationsmittel nur noch wenigstens ein sich unter den Bedingungen der heterogen katalysierten gasphasenkatalytischen Oxidation im wesentlichen inert verhaltendes Verdünnungsgas umfaßt, wobei im kontinuierlichen Betrieb aus dem Produktgasgemisch wenigstens ein Teil der darin enthaltenen im wesentlichen inerten Verdünnungsgasbestandteile abgetrennt und als Bestandteil der Beschickung des Oxidationsreaktors wiederverwendet wird.

Acrylsäure ist eine bedeutende Grundchemikalie, die unter anderem als Monomeres zur Herstellung von Polymerisaten Verwendung findet, die beispielsweise in disperser Verteilung in wäßrigem Medium befindlich als Bindemittel angewendet werden. Acrolein ist ein bedeutendes Zwischenprodukt, beispielsweise für die Herstellung von Glutardialdehyd, Methionin, Folsäure und Acrylsäure.

Es ist allgemein bekannt, Acrylsäure durch heterogen katalysierte Gasphasenoxidation von Propylen mit molekularem Sauerstoff an im festen Aggregatzustand befindlichen Katalysatoren herzustellen (vgl. z.B. DE-A 19 62 431, DE-A 29 43 707, DE-PS 1 205 502, EP-A 257 565, EP-A 253 409, DE-AS 22 51 364, EP-A 117 146, GB-PS 1 450 986 und EP-A 293 224).

Bei den zu verwendenden Katalysatoren handelt es sich normalerweise um Oxidmassen. Die katalytisch aktive Oxidmasse kann neben Sauerstoff lediglich ein anderes Element oder mehr als ein anderes Element (Multielementoxidmassen) enthalten. Besonders häufig kommen als katalytisch aktive Oxidmassen solche zur Anwendung, die mehr als ein metallisches, insbesondere übergangsmetallisches, Element umfassen. In diesem Fall spricht man von Multimetalloxidmassen. Üblicherweise sind die Multielementoxidmassen keine einfachen physikalischen Gemische von Oxiden der elementaren Konstituenten, sondern heterogene Gemische von komplexen Polyverbindungen dieser Elemente.

In der Regel erfolgt die heterogen katalysierte Gasphasenoxidation von Propylen zu Acrylsäure bei erhöhter Temperatur (normalerweise einige hundert °C, typischerweise 200 bis 450°C).

Da die heterogen katalysierte Gasphasenoxidation von Propylen zu Acrylsäure stark exotherm verläuft, führt man sie in zweckmäßiger Weise im Wirbelbett oder in Vielkontaktrohr-Festbettreaktoren durch, durch deren die Kontaktrohre umgebenden Raum ein Wärmeaustauschmittel geleitet wird. Letztere Verfahrensweise ist die bevorzugte (vgl. z.B. DE-A 44 31 957 und DE-A 44 31 949). Der Arbeitsdruck (Absolutdruck) beträgt normalerweise 1 bis 10 bar. Die Zielumsetzung erfolgt während der Verweilzeit des Reaktionsgasgemisches in der Katalysatorbeschickung, durch die es geleitet wird.

Wie dem Fachmann allgemein bekannt ist, läuft die heterogen katalysierte Gasphasen-Partialoxidation von Propylen zu Acrylsäure prinzipiell in zwei längs der Reaktionskoordinate aufeinanderfolgenden Schritten ab, von denen der erste zum Acrolein und der zweite vom Acrolein zur Acrylsäure führt. Aufgrund dieser Inhärenz schließt eine Eignung des erfindungsgemäßen Verfahrens für die gasphasenkatalytisch oxidative Herstellung von Acrylsäure aus Propylen automatisch eine Eignung für die gasphasenkatalytisch oxidative Herstellung von Acrolein aus Propylen ein, da die Acrylsäureherstellung jederzeit auf der Acroleinstufe abgebrochen werden kann. Ferner eröffnet der Reaktionsablauf in zwei zeitlich aufeinanderfolgenden Schritten die Möglichkeit, die gasphasenkatalytisch oxidative Herstellung von Acrylsäure aus Propylen in zwei hintereinander angeordneten Oxidationsstufen auszuführen, wobei in jeder der beiden Oxidationsstufen der zu verwendende oxidische Katalysator in optimierender Weise angepaßt werden kann. So wird für die erste Oxidationsstufe (Propylen → Acrolein) in der Regel ein Katalysator auf der Basis von die Elementkombination Mo-Bi-Fe enthaltenden Multimetalloxiden bevorzugt, während für die zweite Oxidationsstufe (Acrolein → Acrylsäure) normalerweise Katalysatoren auf der Basis von die Elementkombination Mo-V enthaltenden Multimetalloxiden bevorzugt werden. Entsprechende Multimetalloxidkatalysatoren für die beiden Oxidationsstufen sind vielfach vorbeschrieben und dem Fachmann wohlbekannt. Beispielsweise verweist die EP-A 253 409 auf Seite 5 auf entsprechende US-Patente. Günstige Katalysatoren offenbaren auch die DE-A 44 31 957 und die DE-A 44 31 949, insbesondere in Form der Multimetalloxidmassen der allgemeinen Formel I. In der Regel wird das Produktgemisch der ersten Oxidationsstufe ohne Zwischenbehandlung in die zweite Oxidationsstufe überführt. Die einfachste Realisierungsform der beiden Oxidationsstufen bildet daher ein Rohrbündelreaktor innerhalb dessen sich die Katalysatorbeschickung längs der einzelnen Kontaktrohre mit Beendigung des ersten Reaktionsschrittes entsprechend ändert.

Die beiden Oxidationsstufen können jedoch auch in Form eines aus zwei hintereinandergeschalteten Oxidationsreaktoren bestehenden Oxidationsreaktors realisiert sein. In diesem Fall können auch die übrigen Reaktionsbedingungen, z.B. die Reaktionstemperatur, in der jeweiligen Oxidationsstufe in einfacher Weise optimierend angepaßt werden. Zweckmäßigerweise führt man in diesem Fall den für die zweite Oxidationsstufe benötigten molekularen Sauerstoff erst dem zweiten Oxidationsreaktor zu. Prinzipiell ist die heterogen katalysierte Gasphasen-Partialoxidation von Propylen zu Acrylsäure aber auch einstufig realisierbar. In diesem Fall erfolgen beide Reaktionsschritte in einem Oxidationsreaktor, der mit einem Katalysator beschickt ist und die Umsetzung beider Reaktionsschritte katalysiert. Selbstverständlich kann sich auch die Katalysatorbeschickung innerhalb einer Oxidationsstufe längs der Reaktionskoordinate kontinuierlich oder abrupt ändern.

Allen vorgenannten Ausführungsvarianten ist gemein, daß, aufgrund des ausgeprägt exothermen Charakters der Partialoxidation des Propylens, die Oxidationsreaktoren üblicherweise mit einem Gasgemisch beschickt werden, das die Reaktanten molekularer Sauerstoff und Propylen mit einem sich unter den Bedingungen der gasphasenkatalytischen Partialoxidation im wesentlichen inerten Gas verdünnt enthält. Hier werden darunter Verdünnungsgase verstanden, deren Bestandteile unter den Bedingungen der heterogen katalysierten gasphasenkatalytischen Partialoxidation, jeder Bestandteil für sich betrachtet, zu mehr als 95 mol.-%, vorzugsweise zu mehr als 98 mol.-%, unverändert erhalten bleiben. Üblicherweise vereinigt das inerte Verdünnungsgas den größten Volumenanteil der drei Bestandteile des Beschickungsgasgemisches auf sich.

Der vorgenannten Maßnahme steht das Interesse an einer möglichst hohen Raum-Zeit-Ausbeute der gewünschten Zielverbindung entgegen. D.h., es besteht Interesse daran, den Volumenanteil der Reaktionspartner im Beschickungsgasgemisch möglichst hoch zu wählen. Von besonderer Bedeutung ist dabei der Volumenanteil des als Oxidationsmittel zu verwendenden molekularen Sauerstoffs im Beschickungsgasgemisch, wie nachfolgend ausgeführt wird.

So ist es einerseits bezüglich der Stöchiometrie der Partialoxidation zur gewünschten Zielverbindung in der Regel erforderlich, den als Oxidationsmittel verwendeten molekularen Sauerstoff in wenigstens stöchiometrischen oder in überstöchiometrischen Mengen einzusetzen (z.B. aus Gründen der Re-Oxidation der als Katalysator eingesetzten oxidischen Masse sowie zur Minderung von Kohlenstoffabscheidungen).

Andererseits muß der Volumenanteil des als Oxidationsmittel eingesetzten molekularen Sauerstoffs im Beschickungsgemisch aus Sicherheitsgründen unterhalb der sogenannten Sauerstoffgrenzkonzentration liegen.

Unter der Sauerstoffgrenzkonzentration versteht man denjenigen prozentualen Volumenanteil an molekularem Sauerstoff des Beschickungsgasgemisches, bei dessen Unterschreiten unabhängig von der Quantität der Volumenanteile der anderen Bestandteile des Beschickungsgasgemisches (diese Volumenanteile können im kontinuierlichen Betrieb infolge von Störungen in nicht beabsichtigter Weise fluktuieren), nämlich der partiell zu oxidierenden organischen Verbindung sowie dem inerten Verdünnungsgas, eine durch eine örtliche Zündquelle (wie z.B. lokale Überhitzung oder Funkenbildung im Reaktor) eingeleitete Verbrennung der organischen Substanz bei gegebenem Druck und Temperatur des Beschickungsgasgemisches sich in selbigem nicht mehr von der Zündquelle her auszubreiten vermag, so daß die Gefahr einer Explosion ausgeschlossen ist.

Gemäß dem Vorgesagten legt somit die Sauerstoffgrenzkonzentration des Beschickungsgasgemisches den maximalen Volumenanteil des Beschickungsgasgemisches an der partiell zu oxidierenden organischen Verbindung (Propylen) und damit die erreichbare Raum-Zeit-Ausbeute an Zielprodukt (vgl. auch EP-A 257 565, S. 5, Zeilen 36/37) fest.

Selbstverständlich wird die Sauerstoffgrenzkonzentration des Beschickungsgasgemisches von der Art der Bestandteile des Beschickungsgasgemisches wesentlich beeinflußt, weshalb der Wahl des inerten Verdünnungsgases (seiner Zusammensetzung) für die heterogen katalysierte Gasphasen-Partialoxidation von Propylen besondere Bedeutung zukommt.

Die klassischen Verfahren der heterogen katalysierten Gasphasenoxidation von Propylen zu Acrolein und/oder Acrylsäure weisen keine Rückführungsströme an inertem Verdünnungsgas auf und empfehlen in der Regel Wasserdampf und/oder Stickstoff als inertes Verdünnungsgas zur Ausschaltung des explosiven Bereichs (vgl. z.B. US-A 4 147 885, Spalte 1, Zeilen 20 bis 35, DE-A 20 56 614, Seite 2, letzte beiden Zeilen, DE-AS 20 09 172, Spalte 4, Zeilen 40 bis 45, DE-A 22 02 734, S. 4, Zeilen 18 bis 22, DE-A 30 06 894, Seite 6, Zeile 21 und DE-A 24 36 818, Seite 2, Absatz 3, wobei die DE-A 20 56 614 die besondere Eignung von Wasserdampf als inertem Verdünnungsgas auf dessen relativ erhöhte molare Wärmekapazität zurückführt (Seite 4, Absatz 2, Zeile 4), wohingegen die DE-AS 22 51 364 bezüglich der häufigen Verwendung von Stickstoff als inertem Verdünnungsgas den Kostenaspekt (Luft als Quelle des Oxidationsmittels) anführt).

Die DE-A 19 62 431 betrifft ebenfalls ein Verfahren der gasphasenkatalytisch oxidativen Herstellung von Acrylsäure aus Propylen ohne Inertgasrückführungsstrom. Als geeignete inerte Verdünnungsgase nennt die DE-A 19 62 431 Stickstoff, Wasserdampf, Kohlendioxid oder gesättigte Kohlenwasserstoffe.

Die DE-AS 22 51 364 empfiehlt für ein Verfahren der heterogen katalysierten Gasphasen-Partialoxidation von Propylen zu Acrylsäure ohne Inertgasrückführungsstrom Wasserdampf als inertes Verdünnungsgas, dem Stickstoff oder gesättigte Kohlenwasserstoffe wie Methan, Propan oder Butan zugefügt werden können. Die DE-A 14 68 429 empfiehlt für ein Verfahren der heterogen katalysierten Gasphasenoxidation von Propylen zu Acrylsäure ohne Rückführungsströme als inerte Verdünnungsgase Kohlendioxid, Stickstoff, gesättigte Kohlenwasserstoffe oder Wasserdampf, wobei Wasserdampf bevorzugt wird.

Sowohl die Gesamtheit der Ausführungsbeispiele der DE-A 19 62 431 als auch der DE-AS 22 51 364 und der DE-A 14 68 429 umfassen jedoch kein Beispiel, bei dem ein gesättigter Kohlenwasserstoff als inertes Verdünnungsgas auch nur mitverwendet worden wäre.

Die DE-A 30 06 894 betrifft ebenfalls die Problematik, bei einem heterogen katalysierten Gasphasen-Partialoxidationsverfahren des Propylens einerseits das Durchgehen der Reaktion auszuschließen und andererseits eine möglichst hohe Produktivität zu erzielen (S. 2, Zeilen 11 bis 19). Als Lösung empfiehlt sie, das Beschickungsgasgemisch bei geringer Katalysatoraktivität zuzuführen und anschließend längs der Reaktionskoordinate die Katalysatoraktivität sukzessive zu erhöhen.

Als mögliches inertes Verdünnungsgas benennt die DE-A 30 06 894 Stickstoff, Kohlendioxid und/oder Wasserdampf.

Die DT-AS 17 93 302 betrifft ein Verfahren der heterogen katalysierten Gasphasen-Partialoxidation, bei dem als inertes Verdünnungsgas nach Abtrennung des Zielproduktes das die in der Reaktion erzeugten Kohlenoxide und Wasserdampf enthaltende Reaktionsabgas verwendet wird. Die DE-A 20 56 614 spricht ebenfalls die Problematik des Ausschlusses explosionsartiger Verbrennungsprozesse bei der heterogen katalysierten Gasphasen-Partialoxidation von Propylen an (z.B. S. 3, Absatz 2, letzte zwei Zeilen). Um nachteilige Auswirkungen des bevorzugten Verdünnungsgases Wasserdampf zu vermeiden, empfiehlt die DE-A 20 56 614 die von kondensierbaren Gasen weitgehend befreiten Reaktionsabgase unter teilweisem oder vollständigem Ersatz des Wasserdampfes als inerte Verdünnungsgase in den Oxidationsreaktor rückzuführen und gleichzeitig das Beschickungsgasgemisch bei niederer Katalysatoraktivität zuzuführen und anschließend die Katalysatoraktivität längs der Reaktionskoordinate sukzessive zu erhöhen. Da das Oxidationsmittel "molekularer Sauerstoff" als Bestandteil von Luft zugeführt wird, sind die effektiven inerten Verdünnungsgase bei der Verfahrensweise der DE-A 20 56 614 im wesentlichen Stickstoff und Kohlendioxid. Die Verfahrensweise der DE-A 24 36 818 entspricht hinsichtlich der verwendeten inerten Verdünnungsgase im wesentlichen jener der DE-A 20 56 614. Das gleiche trifft auf die US-A 4 147 885 zu. Die DE-A 27 29 841 betrifft ein Verfahren der heterogen katalysierten Gasphasenoxidation von Propylen zu Acrylsäure, das aufgrund der Verwendung eines speziellen Oxidationskatalysators die Möglichkeit eröffnet, anstelle von Wasserdampf als inertes Verdünnungsmittel ein Gemisch aus CO, CO₂, Stickstoff und Argon, das aus dem Produktgasgemisch der heterogen katalysierten Partialoxidation abgetrennt und in das Beschickungsgasgemisch rückgeführt wird, zu verwenden.

Die EP-B 253 409 (vgl. insbesondere S. 5, erste drei Zeilen) und die EP-A 257 565 lehren im Hinblick auf die Vermeidung eines Explosionsrisikos bei der heterogen katalysierten Gasphasen-Partialoxidation des Propylens die Verwendung von solchen inerten Verdünnungsgasen, die eine erhöhte molare Wärmekapazität Cp aufweisen. Als bevorzugt werden dabei z.B. auf Seite 4, Zeilen 47 ff der EP-B 253 409 sowie auf S. 5, Zeilen 26 ff der EP-A 257 565 Gemische aus Stickstoff, CO₂, Methan, Ethan, Propan und Wasserdampf empfohlen. Neben den genannten Gasen können aber auch noch Helium, Argon, andere gesättigte Kohlenwasserstoffgase, N₂O und Kohlenmonoxid enthalten sein. Als für die Wirkung des inerten Verdünnungsgases wesentlich wird lediglich dessen mittlere molare Wärmekapazität erachtet.

So besteht das inerte Verdünnungsgas des Beschickungsgasgemisches in allen Ausführungsbeispielen zu mehr als 55 Vol.-% aus N2. Ferner empfehlen die EP-B 253 409 und die EP-A 257 565 die im Produktgasgemisch enthaltenen inerten Verdünnungsgase zumindest teilweise in das Beschickungsgasgemisch zurückzuführen.

Das G.B. Patent Nr. 14 50 986 empfiehlt, insbesondere aufgrund seiner erhöhten Wärmeaufnahmefähigkeit, die Verwendung von Kohlendioxid als im wesentlichen alleiniges inertes Verdünnungsgas zur Vermeidung der Explosionsgefahr bei der gasphasenkatalytisch oxidativen Herstellung von Acrylsäure aus Propylen.

Die EP-A 293 224 betrifft ein Verfahren der heterogen katalysierten Gasphasenoxidation von Propylen zu Acrylsäure, bei dem zur Gewährleistung einer sicheren Verfahrensdurchführung die Verwendung eines Kohlendioxid, Wasserdampf und 1 bis 5 C-Atome aufweisende gesättigte Kohlenwasserstoffe enthaltenden Gasgemisches als Inertgas empfohlen wird (S. 3, Zeilen 9 und 20 der EP-A 293 224). Als wesentlich für die Wirksamkeit des seitens der EP-A 293 224 empfohlenen Inertgasgemisches erachtet die EP-A 293 224 das Beisein von Kohlenoxiden in erhöhten Konzentrationen (Seite 3, Zeile 57) sowie eine erhöhte molare Wärmekapazität des Inertgasgemisches (Seite 3, Zeile 57). Als weiteren besonderen Vorteil der ihrerseits empfohlenen Verfahrensweise erachtet die EP-A 293 224 die Tatsache, daß das zu verwendende Inertgasgemisch zu erheblichen Teilen aus dem Produktgasgemisch der Partialoxidation rekrutiert werden kann. In allen Ausführungsbeispielen umfaßt das im Beschickungsgasgemisch verwendete Inertgasgemisch Wasserdampf und CO₂ in einer Gesamtmenge von wenigstens 15 Vol.-%, bezogen auf das Inertgasgemisch.

Die EP-A 117 146 betrifft ein Verfahren der heterogen katalysierten Gasphasen-Partialoxidation von Propylen zu Acrylsäure, wobei das Propylen durch heterogen katalysierte Dehydrierung von Propan erzeugt wird. Als besonderen Vorteil stellt die EP-A 117 146 heraus, daß man das Produktgemisch der Propandehydrierung ohne Zwischenbehandlung in die Oxidationsstufe überführen und die sich dabei inert verhaltenden Bestandteile anschließend in die Propandehydrierungsstufe rückführen kann. In entsprechender Weise umfaßt das Beschickungsgasgemisch in allen Ausführungsbeispielen ein inertes Verdünnungsgas, das zu mehr als 15 Vol.-% aus Wasserdampf besteht.

Nachteilig an den kontinuierlich betriebenen Verfahren der heterogen katalysierten Gasphasenoxidation von Propylen zu Acrylsäure des Standes der Technik ist, daß die damit einhergehenden inerten Verdünnungsgase der Beschickungsgasgemische hinsichtlich der daran gekoppelten Sauerstoffgrenzkonzentrationen nicht zu befriedigen vermögen.

Ein weiterer Nachteil der Verfahrensweise der EP-A 117 146 besteht darin, daß sie in notwendiger Weise an Propan als Propylen-Quelle geknüpft ist.

Aufgabe der vorliegenden Erfindung war daher ein Verfahren der kontinuierlich betriebenen heterogen katalysierten Gasphasenoxidation von Propylen zu Acrolein, Acrylsäure oder deren Gemisch in einem Oxidationsreaktor, dessen Beschickungsgasgemisch neben Propylen und molekularem Sauerstoff als Oxidationsmittel nur noch wenigstens ein sich unter den Bedingungen der heterogen katalysierten Gasphasenoxidation im wesentlichen inert verhaltendes Verdünnungsgas umfaßt, wobei im kontinuierlichen Betrieb aus dem Produktgasgemisch wenigstens ein Teil der darin enthaltenen im wesentlichen inerten Verdünnungsgasbestandteile abgetrennt und als Bestandteil der Beschickung des Oxidationsreaktors wiederverwendet wird, das die aufgeführten Nachteile der Verfahren des Standes der Technik nicht aufweist.

Demgemäß wurde ein Verfahren der kontinuierlich betriebenen heterogen katalysierten Gasphasenoxidation von Propylen zu Acrolein, Acrylsäure oder deren Gemisch in einem Oxidationsreaktor, dessen Beschickungsgasgemisch neben Propylen und molekularem Sauerstoff als Oxidationsmittel nur noch wenigstens ein sich unter den Bedingungen der heterogen katalysierten Gasphasenoxidation im wesentlichen inert verhaltendes Verdünnungsgas umfaßt, wobei im kontinuierlichen Betrieb aus dem Produktgasgemisch wenigstens ein Teil der darin enthaltenen im wesentlichen inerten Verdünnungsgasbestandteile abgetrennt und als Bestandteil der Beschickung des Oxidationsreaktors wiederverwendet wird, gefunden, das dadurch gekennzeichnet ist, daß das im wesentlichen inerte Verdünnungsgas des Beschickungsgasgemisches im kontinuierlichen Betrieb zu mehr als 85 Vol.-%, vorzugsweise zu wenigstens 90 Vol.-%, besser zu wenigstens 95 Vol.-%, noch besser zu wenigstens 97 Vol.-%, vorteilhaft zu wenigstens 98 Vol.-%, bevorzugt zu wenigstens 99 Vol.-% und am besten zu 100 Vol.-% aus wenigstens einem 1 bis 5 C-Atome aufweisenden gesättigten Kohlenwasserstoff besteht.

Als von 1 bis 5 C-Atome aufweisenden gesättigten Kohlenwasserstoffen verschiedene Bestandteile des inerten Verdünnungsgases des Beschickungsgasgemisches kommen u.a. CO₂, CO, N₂, H₂O, höhere gesättigte Kohlenwasserstoffe und/oder Edelgase wie He und Ar in Betracht.

Mit Vorteil liegt die mittlere molare spezifische Wärme Cₚ des inerten Verdünnungsgases des Beschickungsgasgemisches im Bereich 10 bis 50, vorzugsweise 10 bis 35 cal/molK (bezogen auf 1 atm. u. 300°C).

Vorzugsweise enthält das inerte Verdünnungsgas des Beschickungsgasgemisches keinen Wasserdampf.

Als 1 bis 5 C-Atome aufweisende gesättigte Kohlenwasserstoffe kommen in Betracht Methan, Ethan, Propan, n-Butan, iso-Butan, n-Pentan, iso-Pentan, neo-Pentan und Mischungen der genannten Kohlenwasserstoffe. Unter den vorgenannten Kohlenwasserstoffen sind Methan, Propan und deren Gemische bevorzugt zu verwendende Verdünnungsgasbestandteile. Dies gilt vor allem für Methan, das sich bei einer Reaktionsführung im Rohrbündelreaktor gleichzeitig in besonders vorteilhafter Weise zur Vermeidung lokaler Überhitzungen ("hot spots") längs der Kontaktrohre eignet und sich in besonders hohem Ausmaß inert verhält. Propan erweist sich insofern als günstig, als es sich bei geringfügig nicht inertem Verhalten normalerweise überwiegend zu Propylen, Acrolein und/oder Acrylsäure umsetzt.

Die hier gegebene Lehre ist das Ergebnis ausführlicher und systematischer Forschungsarbeiten. Sie fußt auf der Erkenntnis, daß für die Sauerstoffgrenzkonzentration eines aus molekularem Sauerstoff, inertem Verdünnungsgas und Propylen bestehenden Beschickungasgemisches (längs der Reaktionskoordinate nimmt der Anteil an molekularem Sauerstoff jeweils ab) weniger die molare Wärmekapazität Cp des inerten Verdünnungsgases als vielmehr die Eigenschaft des inerten Verdünnungsgases, im wesentlichen selbst eine brennbare niedere organische Verbindung zu sein, von Relevanz ist, wobei das gleichzeitige Erfordernis der Inertheit die Beschränkung auf niedere gesättigte Kohlenwasserstoffe bedingt.

Das Merkmal brennbar bringt dabei zum Ausdruck, daß es sich um Verbindungen handelt, deren bei einem Ausgangsdruck von 1 bar und einer Ausgangstemperatur von 50 bis 100°C befindliche Mischungen mit Luft eine obere und eine untere Explosionsgrenze (Entzündungsgrenze) aufweisen, wobei sich die Ermittlung der Explosionsgrenzen auf eine Bestimmung in der Standard Apparatur gemäß W. Berthold et al. in Chem.-Ing. Tech. 56 (1984) Nr. 2, S. 126-127 bezieht (N₂, CO₂ und H₂O sind beispielsweise keine brennbaren Verbindungen).

Unter Explosionsgrenzen werden dabei nachfolgende Grenzwerte gemäß DIN 51 649 verstanden:
In einem Gemisch aus Luft und einem brennbaren Gas ist die Geschwindigkeit, mit der sich unter vorgegebenen Ausgangsbedingungen eine durch eine örtliche Zündquelle (z.B. glühender Platindraht) eingeleitete Verbrennung (Entzündung, Explosion) ausbreitet, vom Gehalt an brennbarem Gas abhängig. Sie ist bei einem bestimmten Gehalt am größten. Sowohl bei Verringerung als auch bei Erhöhung des Gehaltes an brennbarem Gas wird die Verbrennungsgeschwindigkeit kleiner, bis sich schließlich die Verbrennungsreaktion bei einem unteren und einem oberen Grenzwert für den Gehalt an brennbarem Gas gerade nicht mehr von der Zündquelle her ausbreitet. Diese beiden Grenzwerte sind die untere Explosionsgrenze und die obere Explosionsgrenze, der dazwischen liegende Bereich des Gehalts an brennbarem Gas ist der Explosionsbereich (Entzündungsbereich).

Je höher der Anteil an den niederen gesättigten Kohlenwasserstoffen im inerten Verdünnungsgas des Beschickungsgasgemisches der heterogen katalysierten Gasphasenoxidation von Propylen ist, desto sicherer läßt sich diese auch bei erhöhten Volumenanteilen der Reaktanten durchführen.

Ein besonderer Gesichtspunkt des erfindungsgemäßen Verfahrens ist, daß die erfindungsgemäß als inerte Verdünnungsgasbestandteile des Beschickungsgasgemisches zu verwendenden niederen gesättigten Kohlenwasserstoffe im Unterschied zu den im Stand der Technik bevorzugten inerten Verdünnungsgasbestandteilen wie N₂, H₂O und CO₂ aufgrund ihrer Brennbarkeit Wertstoffe darstellen, die beispielsweise zur Energieerzeugung verwendet werden können. Wesentliches Merkmal des erfindungsgemäßen Verfahrens ist daher, daß wenigstens ein Teil der im Produktgasgemisch als inerte Verdünnungsgasbestandteile enthaltenen 1 bis 5 C-Atome aufweisenden gesättigten Kohlenwasserstoffe aus selbigem abgetrennt und als Bestandteil eines neuen Beschickungsgasgemisches in die Beschickung des Oxidationsreaktors rückgeführt (wiederverwendet) wird.

Vorzugsweise werden im kontinuierlichen Betrieb wenigstens 50 Vol.-% der im Produktgasgemisch enthaltenen 1 bis 5 C-Atome aufweisenden gesättigten Kohlenwasserstoffe rückgeführt. Besonders bevorzugt beträgt dieser Anteil wenigstens 75 Vol.-%, besser wenigstens 90 oder 95 Vol.-% und noch besser wenigstens 98 oder 99 oder 100 Vol.-%.

Vorgenannte Rückführung ist Voraussetzung für die wirtschaftliche Durchführbarkeit des erfindungsgemäßen Verfahrens. Dabei ist zu beachten, daß im Rahmen dieser Rückführung der 1 bis 5 C-Atome aufweisenden gesättigten Kohlenwasserstoffe in der Regel nicht nur eine Abtrennung derselben vom im Produktgasgemisch enthaltenen Zielprodukt, sondern auch von darin enthaltenen Nebenprodukten erfolgen muß (zumindest teilweise). Dies gilt auch dann, wenn es sich bei letzteren um bezüglich der Gasphasenoxidation des Propylens inerte Gase wie CO₂ oder H₂O handelt, da sich diese ansonsten im Verlauf des kontinuierlichen Betriebs im Verdünnungsgas des Beschickungsgasgemisches gegebenenfalls anreichern. Selbstverständlich kann im Rahmen des erfindungsgemäßen Verfahrens auch im Produktgasgemisch noch enthaltenes nicht umgesetztes Ausgangs- und/oder Zwischenprodukt aus selbigem abgetrennt und in den Oxidationsreaktor rückgeführt werden.

Die dabei anzuwendenden Trennverfahren wie fraktionierte Kondensation oder Absorptions-, Extraktionsverfahren sind dem Fachmann bekannt und bedürfen keiner näheren Erläuterung. Die Aufarbeitung und Abtrennung des erwünschten Zielproduktes erfolgt ebenfalls in an sich bekannter Weise.

Als Quelle für den im Rahmen des erfindungsgemäßen Verfahrens als Oxidationsmittel benötigten molekularen Sauerstoff ist Luft nur in beschränktem Ausmaß geeignet, da molekularer Sauerstoff in Luft nur mit N₂ vergesellschaftet vorkommt.

D.h. vorzugsweise wird man den für das erfindungsgemäße Verfahren benötigten Sauerstoff einer im wesentlichen reinen Sauerstoffquelle entnehmen.

Der Unterschied des erfindungsgemäßen Verfahrens zu den Verfahren des Standes der Technik besteht zusammenfassend im wesentlichen darin, daß bei Anwendung von ansonsten identischen Reaktionsbedingungen bei Verwendung des erfindungsgemäßen Anteils an 1 bis 5 C-Atome aufweisenden gesättigten Kohlenwasserstoffen am im Beschickungsgasgemisch eingesetzten inerten Verdünnungsgas die kontinuierlich betriebene heterogen katalysierte Gasphasenoxidation von Propylen zu Acrolein, Acrylsäure oder deren Gemisch, insbesondere bei erhöhten Reaktantenvolumenanteilen im Beschickungsgasgemisch, unter erhöhter Sicherheit durchführbar ist (auch die Beschickung mit molekularem Sauerstoff kann im kontinuierlichen Betrieb infolge von unbeabsichtigten Störungen fluktuieren), was die Grundlage für erhöhte Raum-Zeit-Ausbeuten bildet. Nachfolgend wird dies an einigen Ausführungsbeispielen ausgewiesen. Abschließend sei festgehalten, daß für die heterogen katalysierte Gasphasenoxidation von Propylen zu Acrolein im Beschickungsgasgemisch vorzugsweise eine Propylen/molekularer Sauerstoff Beschickung gewählt wird, deren Volumenanteile sich wie 1 (Propylen):1 bis 3 (molekularer Sauerstoff), vorzugsweise wie 1 (Propylen):1,5 bis 2 (molekularer Sauerstoff), verhalten. Wie bereits erwähnt, wirkt sich der Sauerstoffüberschuß vorteilhaft auf die Kinetik der Gasphasenoxidation sowie auf die Lebensdauer des Katalysators aus. Die thermodynamischen Verhältnisse werden dadurch im wesentlichen nicht beeinflußt, da die erfindungsgemäße heterogen katalysierte Gasphasen-Partialoxidation kinetischer Kontrolle unterliegt. Vorgenannte Propylen/molekularer Sauerstoff Beschickung wird mit Vorteil auch für die zweistufige Gasphasenoxidation von Propylen zu Acrylsäure (z.B. in einem aus zwei hintereinandergeschalteten Oxidationsreaktoren bestehenden Oxidationsreaktor) gewählt. In der Regel wird dabei das die erste Stufe (den ersten Oxidationsreaktor) verlassende Produktgasgemisch ohne Zwischenbehandlung in den zweiten Oxidationsreaktor überführt. Zweckmäßigerweise führt man der zweiten Oxidationsstufe (dem zweiten Oxidationsreaktor) zusätzlich molekularen Sauerstoff als Oxidationsmittel zu. Die Menge an zusätzlich zugeführtem molekularen Sauerstoff wird vorzugsweise so gewählt, daß auch das Beschickungsgasgemisch der zweiten Oxidationsstufe (des zweiten Oxidationsreaktors) eine wenigstens stöchiometrische bis etwa dreifach stöchiometrische Menge an O₂ umfaßt. Dabei wird man den Sauerstoff vorzugsweise ebenfalls einer im wesentlichen reinen Sauerstoffquelle entnehmen. Die Sicherheit für die zweite Oxidationsstufe (den zweiten Oxidationsreaktor) kann man zusätzlich dadurch erhöhen, daß man aus dem die erste Oxidationsstufe (den ersten Oxidationsreaktor) verlassenden Produktgasgemisch in der ersten Oxidationsstufe als Nebenprodukte gebildeten Wasserdampf und CO₂ abtrennt, bevor man es in die zweite Oxidationsstufe (in den zweiten Oxidationsreaktor) überführt. Es sei festgehalten, daß gemäß der erfindungsgemäßen Verfahrensweise Beschickungsgasgemische sicherer handhabbar sind, deren Propylenbeschickung > 30 Vol.-% (bei günstiger Wahl des inerten Verdünnungsgases bis zu 40 oder 45 Vol.-%), bezogen auf das Beschickungsgasgemisch beträgt.

Günstige Beschickungsgasgemische sind auch solche, die im kontinuierlichen Betrieb umfassen

| | |
|---|---|
| 15 bis 30 Vol.-% | Propylen, |
| 20 bis 40 Vol.-% | Sauerstoff und |
| 30 bis 65 Vol.-% | erfindungsgemäßes inertes Verdünnungsgas. |

### Beispiele (Einfluß der Brennbarkeit der inerten Verdünnungsgasbestandteile auf die Sauerstoffgrenzkonzentration)

Ermittlung der Sauerstoffgrenzkonzentration von bei einer Ausgangstemperatur von 250°C und einem Ausgangsdruck von 1 bar befindlichen Beschickungsgasgemischen, bestehend aus Propylen (partiell zu oxidierende organische Verbindung), molekularem Sauerstoff (Oxidationsmittel) und einem bezüglich einer heterogen katalysierten Gasphasen-Partialoxidation des Propylens zu Acrylsäure inerten Verdünnungsgas.

### Allgemeine Versuchsdurchführung:

Die Versuche wurden in einem geschlossenen kugelförmigen 51-Hochdruckbehälter aus Edelstahl durchgeführt. Die Erzeugung des Gasgemisches in dem anfangs evakuierten Hochdruckbehälter erfolgte nach der Partialdruckmethode. Nach einer Mischzeit von 10 min mittels eines Magnetrührwerkes wurde versucht, das Gasgemisch mittels eines durchschmelzenden Platindrahtes zu zünden. Eine dadurch eventuell ausgelöste selbständige Ausbreitung einer Reaktionsfront (Explosion) wurde über den zeitlichen Anstieg des Behälterinnendruckes (Messung mit piezoelektrischem Druckaufnehmer) und über die Erhöhung der Temperatur im Behälter detektiert.

Ergebnisse (die zugrunde gelegten spezifischen molaren Wärmen Cp fußen auf den Daten aus "Ihsan Barin, Thermochemical Data of Pure Substances, Part I u. Part II, VCH Verlagsgesellschaft, Weinheim, Zweite Auflage, 1993", wobei bei Gasgemischen ideales Gasverhalten angenommen wurde):
a) Ausschließliche Verwendung von Methan als inertes brennbares Verdünnungsgas. D.h. das inerte Verdünnungsgas bestand ausschließlich aus brennbaren Bestandteilen. Die spezifische molare Wärme Cp von Methan beträgt unter den vorgegebenen Bedingungen 47,5 J/mol·K. Die ermittelte Sauerstoffgrenzkonzentration beträgt 32 Vol.-%.
   D.h. in einem Gemisch aus Propylen, molekularem O₂ und Methan als Inertgas, das sich bei 250°C und 1 bar befindet, vermag sich eine lokale Entzündung (Explosion) unabhängig von der speziellen Gemischzusammensetzung dann nicht mehr selbständig auszubreiten, wenn der Volumenanteil an O₂ im Gesamtgemisch < 32 Vol.-% beträgt. D.h. in einem bei 1 bar und 250°C befindlichen Gemisch aus 31 Vol.-% O₂, 20 Vol.-% Propylen und 49 Vol.-% Methan vermag sich eine lokale Entzündung nicht mehr selbständig auszubreiten.
b) Verwendung eines 3,2 (Propan) : 96,8 (CO₂)-Gemisches (Verhältnis der Volumenanteile) aus Propan und Kohlendioxid als inertes Verdünnungsgas. D.h. das inerte Verdünnungsgas bestand fast ausschließlich aus nicht brennbarem Verdünnungsgas. Die Zusammensetzung des Inertgasgemisches wurde so gewählt, damit es unter den vorgegebenen Bedingungen ebenfalls ein Cp von 47,5 J/mol·K aufweist. Die ermittelte Sauerstoffgrenzkonzentration beträgt lediglich 15 Vol.-%.
   D.h. in einem unter entsprechenden Bedingungen wie in a) befindlichen Gemisch aus 31 Vol.-% O₂, 20 Vol.-% Propylen und 49 Vol.-% des inerten Verdünnungsgases breitet sich eine lokale Entzündung selbständig aus.
c) Verwendung eines 48,3 (Propan) : 51,7 (Methan)-Gemisches (Verhältnis der Volumenanteile) aus Propan und Methan als inertes Verdünnungsgas. D.h. das inerte Verdünnungsgas bestand ausschließlich aus brennbaren Bestandteilen. Die spezifische molare Wärme Cp dieses Gemisches beträgt unter den vorgegebenen Bedingungen = 80,8 J/mol·K. Die ermittelte Sauerstoffgrenzkonzentration beträgt 37 Vol.-%.
d) Verwendung eines 50 (Propan) : 50 (CO₂)-Gemisches (Verhältnis der Volumenanteile) aus Propan und Kohlendioxid als inertes Verdünnungsgas. D.h. das inerte Verdünnungsgas enthielt auch nicht brennbare Bestandteile. Die Zusammensetzung des Inertgasgemisches wurde so gewählt, damit es unter den vorgegebenen Bedingungen ebenfalls ein Cp von 80,8 J/mol·K aufweist.
   Die ermittelte Sauerstoffgrenzkonzentration beträgt lediglich 34 Vol.-%. D.h. trotz signifikant höherem Cp-Wert des inerten Verdünnungsgases im Vergleich zu a), liegt die Sauerstoffgrenzkonzentration nur bei einem vergleichbaren Volumenanteil wie bei a).

## Patentansprüche

1. Verfahren der kontinuierlich betriebenen heterogen katalysierten Gasphasenoxidation von Propylen zu Acrolein, Acrylsäure oder deren Gemisch in einem Oxidationsreaktor, dessen Beschickungsgasgemisch neben Propylen und molekularem Sauerstoff als Oxidationsmittel nur noch wenigstens ein sich unter den Bedingungen der heterogen katalysierten Gasphasenoxidation im wesentlichen inert verhaltendes Verdünnungsgas umfaßt, wobei im kontinuierlichen Betrieb aus dem Produktgasgemisch wenigstens ein Teil der darin enthaltenen im wesentlichen inerten Verdünnungsgasbestandteile abgetrennt und als Bestandteil der Beschickung des Oxidationsreaktors wiederverwendet wird, dadurch gekennzeichnet, daß das im wesentlichen inerte Verdünnungsgas des Beschickungsgasgemisches im kontinuierlichen Betrieb zu mehr als 85 Vol.-% aus wenigstens einem 1 bis 5 C-Atome aufweisenden gesättigten Kohlenwasserstoff besteht.

2. Verfahren nach Anspruch 1, dadurch gekennzeichnet, daß das im wesentlichen inerte Verdünnungsgas des Beschickungsgasgemisches im kontinuierlichen Betrieb zu wenigstens 90 Vol.-% aus wenigstens einem 1 bis 5 C-Atome aufweisenden gesättigten Kohlenwasserstoff besteht.

3. Verfahren nach Anspruch 1, dadurch gekennzeichnet, daß das im wesentlichen inerte Verdünnungsgas des Beschickungsgasgemisches im kontinuierlichen Betrieb zu wenigstens 95 Vol.-% aus wenigstens einem 1 bis 5 C-Atome aufweisenden gesättigten Kohlenwasserstoff besteht.

4. Verfahren nach Anspruch 1, dadurch gekennzeichnet, daß das im wesentlichen inerte Verdünnungsgas des Beschickungsgasgemisches im kontinuierlichen Betrieb zu wenigstens 97 Vol.-% aus wenigstens einem 1 bis 5 C-Atome aufweisenden gesättigten Kohlenwasserstoff besteht.

5. Verfahren nach Anspruch 1, dadurch gekennzeichnet, daß das im wesentlichen inerte Verdünnungsgas des Beschickungsgasgemisches im kontinuierlichen Betrieb zu wenigstens 98 Vol.-% aus wenigstens einem 1 bis 5 C-Atome aufweisenden gesättigten Kohlenwasserstoff besteht.

6. Verfahren nach Anspruch 1, dadurch gekennzeichnet, daß das im wesentlichen inerte Verdünnungsgas des Beschickungsgasgemisches im kontinuierlichen Betrieb zu wenigstens 99 Vol.-% aus wenigstens einem 1 bis 5 C-Atome aufweisenden gesättigten Kohlenwasserstoff besteht.

7. Verfahren nach Anspruch 1, dadurch gekennzeichnet, daß das im wesentlichen inerte Verdünnungsgas des Beschickungsgasgemisches im kontinuierlichen Betrieb zu 100 Vol.-% aus wenigstens einem 1 bis 5 C-Atome aufweisenden gesättigten Kohlenwasserstoff besteht.

8. Verfahren nach Anspruch 1, dadurch gekennzeichnet, daß der gesättigte Kohlenwasserstoff zu wenigstens 50 mol.-% Methan ist.

9. Verfahren nach Anspruch 1, dadurch gekennzeichnet, daß der gesättigte Kohlenwasserstoff zu wenigstens 50 mol-% Propan ist.

10. Verfahren nach Anspruch 1, dadurch gekennzeichnet, daß die kontinuierlich betriebene heterogen katalysierte Gasphasenoxidation von Propylen zu Acrylsäure in einem aus zwei hintereinandergeschalteten Oxidationsreaktoren bestehenden Oxidationsreaktor durchgeführt wird, wobei man das Beschikkungsgasgemisch im ersten Oxidationsreaktor aufgibt und in diesem Propylen im wesentlichen bis zum Acrolein oxidiert, anschließend das Produktgasgemisch des ersten Oxidationsreaktors ohne Zwischenbehandlung in den zweiten Oxidationsreaktor leitet und letzterem die für die Weiteroxidation des Acroleins zur Acrylsäure erforderliche Menge an molekularem Sauerstoff zuführt.

## Claims

1. A process for the continuous heterogeneously catalyzed gas-phase oxidation of propylene to acrolein, acrylic acid or a mixture thereof in an oxidation reactor whose feed gas mixture comprises, apart from propylene and molecular oxygen as oxidant, only at least one diluent gas which is essentially inert under the conditions of the heterogeneously catalyzed gas-phase oxidation, where, in continuous operation, at least a part of the essentially inert diluent gas constituents present in the product gas mixture is separated off therefrom and is reused as a constituent of the feed to the oxidation reactor, wherein the essentially inert diluent gas of the feed gas mixture in continuous operation comprises more than 85 % by volume of at least one saturated hydrocarbon having from 1 to 5 carbon atoms.

2. A process as claimed in claim 1, wherein the essentially inert diluent gas of the feed gas mixture in continuous operation comprises at least 90 % by volume of at least one saturated hydrocarbon having from 1 to 5 carbon atoms.

3. A process as claimed in claim 1, wherein the essentially inert diluent gas of the feed gas mixture in continuous operation comprises at least 95 % by volume of at least one saturated hydrocarbon having from 1 to 5 carbon atoms.

4. A process as claimed in claim 1, wherein the essentially inert diluent gas of the feed gas mixture in continuous operation comprises at least 97 % by volume of at least one saturated hydrocarbon having from 1 to 5 carbon atoms.

5. A process as claimed in claim 1, wherein the essentially inert diluent gas of the feed gas mixture in continuous operation comprises at least 98 % by volume of at least one saturated hydrocarbon having from 1 to 5 carbon atoms.

6. A process as claimed in claim 1, wherein the essentially inert diluent gas of the feed gas mixture in continuous operation comprises at least 99 % by volume of at least one saturated hydrocarbon having from 1 to 5 carbon atoms.

7. A process as claimed in claim 1, wherein the essentially inert diluent gas of the feed gas mixture in continuous operation consists of 100 % by volume of at least one saturated hydrocarbon having from 1 to 5 carbon atoms.

8. A process as claimed in claim 1, wherein the saturated hydrocarbon comprises at least 50 mol % of methane.

9. A process as claimed in claim 1, wherein the saturated hydrocarbon comprises at least 50 mol % of propane.

10. A process as claimed in claim 1, wherein the continuous heterogeneously catalyzed gas-phase oxidation of propylene to acrylic acid is carried out in an oxidation reactor comprising two oxidation reactors connected in series with the feed gas mixture being fed to the first oxidation reactor and propylene being oxidized essentially to acrolein in this reactor, the product gas mixture of the first oxidation reactor subsequently being conveyed without intermediate treatment to the second oxidation reactor and the latter being fed with the amount of molecular oxygen required for the further oxidation of the acrolein to acrylic acid.

## Revendications

1. Procédé d'oxydation en phase gazeuse du propylène en acroléine, acide acrylique ou mélange de ceux-ci, avec catalyse hétérogène, fonctionnant en continu, dans un réacteur d'oxydation dont le mélange gazeux d'alimentation ne comprend, outre du propylène et de l'oxygène moléculaire en tant qu'oxydant, qu'au moins un gaz de dilution qui se comporte de façon pratiquement inerte dans les conditions de l'oxydation en phase gazeuse à catalyse hétérogène, dans le fonctionnement en continu au moins une partie des composants du gaz de dilution pratiquement inerte contenus dans le mélange gazeux produit étant séparée de celui-ci et réutilisée en tant que composant de l'alimentation du réacteur d'oxydation, caractérisé en ce que le gaz de dilution pratiquement inerte du mélange gazeux d'alimentation dans le fonctionnement en continu consiste à raison de plus de 85 % en volume en au moins un hydrocarbure saturé comportant de 1 à 5 atomes de carbone.

2. Procédé selon la revendication 1, caractérisé en ce que le gaz de dilution pratiquement inerte du mélange d'alimentation dans le fonctionnement en continu consiste à raison d'au moins 90 % en volume en au moins un hydrocarbure saturé comportant de 1 à 5 atomes de carbone.

3. Procédé selon la revendication 1, caractérisé en ce que le gaz de dilution pratiquement inerte du mélange gazeux d'alimentation dans le fonctionnement en continu consiste à raison d'au moins 95 % en volume en au moins un hydrocarbure saturé comportant de 1 à 5 atomes de carbone.

4. Procédé selon la revendication 1, caractérisé en ce que le gaz de dilution pratiquement inerte du mélange gazeux d'alimentation dans le fonctionnement en continu consiste à raison d'au moins 97 % en volume en au moins un hydrocarbure saturé comportant de 1 à 5 atomes de carbone.

5. Procédé selon la revendication 1, caractérisé en ce que le gaz de dilution pratiquement inerte du mélange gazeux d'alimentation dans le fonctionnement en continu consiste à raison d'au moins 98 % en volume en au moins un hydrocarbure saturé comportant de 1 à 5 atomes de carbone.

6. Procédé selon la revendication 1, caractérisé en ce que le gaz de dilution pratiquement inerte du mélange gazeux d'alimentation dans le fonctionnement en continu consiste à raison d'au moins 99 % en volume en au moins un hydrocarbure saturé comportant de 1 à 5 atomes de carbone.

7. Procédé selon la revendication 1, caractérisé en ce que le gaz de dilution pratiquement inerte du mélange gazeux d'alimentation dans le fonctionnement en continu consiste à raison de 100 % en volume en au moins un hydrocarbure saturé comportant de 1 à 5 atomes de carbone.

8. Procédé selon la revendication 1, caractérisé en ce que l'hydrocarbure saturé est à raison d'au moins 50 % en moles le méthane.

9. Procédé selon la revendication 1, caractérisé en ce que l'hydrocarbure saturé est à raison d'au moins 50 % en moles le propane.

10. Procédé selon la revendication 1, caractérisé en ce que l'on effectue l'oxydation en phase gazeuse du propylène en acide acrylique, avec catalyse hétérogène, fonctionnant en continu, dans un réacteur d'oxydation constitué de deux réacteurs d'oxydation disposés l'un derrière l'autre, en envoyant le mélange gazeux d'alimentation dans le premier réacteur d'oxydation et en oxydant dans celui-ci le propylène pratiquement jusqu'à l'acroléine, puis en envoyant le mélange gazeux produit du premier réacteur d'oxydation, sans traitement intermédiaire, dans le second réacteur d'oxydation, et en envoyant dans ce dernier la quantité d'oxygène moléculaire requise pour l'oxydation plus poussée de l'acroléine en acide acrylique.
